(19) Europäisches Patentamt
European Patent Office
Office européen des brevets

(11) **EP 3 635 796 B1**

(12) **EUROPEAN PATENT SPECIFICATION**

(45) Date of publication and mention of the grant of the patent:
**20.03.2024 Bulletin 2024/12**

(21) Application number: **18731532.0**

(22) Date of filing: **25.05.2018**

(51) International Patent Classification (IPC):
***C07D 487/22*** *(2006.01)* ***C01B 32/152*** *(2017.01)*
***H10K 85/30*** *(2023.01)* ***H10K 85/20*** *(2023.01)*

(52) Cooperative Patent Classification (CPC):
**C07D 487/22; C01B 32/152; H10K 85/211; H10K 85/30; H10K 85/381;** H10K 10/484; Y02E 10/549

(86) International application number:
**PCT/IN2018/050334**

(87) International publication number:
**WO 2018/216035 (29.11.2018 Gazette 2018/48)**

(54) **AN ASSEMBLY OF PORPHYRIN-FULLERENE AND USE THEREOF**

ZUSAMMENSETZUNG VON PORPHYRINFULLEREN UND IHRE VERWENDUNG

ASSEMBLAGE DE PORPHYRINE-FULLERÈNE ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.05.2017 IN 201711018553**

(43) Date of publication of application:
**15.04.2020 Bulletin 2020/16**


(73) Proprietor: **Council of Scientific and Industrial Research**
**New Delhi 110001 (IN)**

(72) Inventors:
- **SUKUMARAN, Santhosh Babu**
  **Pune**
  **Maharashtra 411008 (IN)**
- **., Goudappagouda**
  **Pune**
  **Maharashtra 411008 (IN)**

(74) Representative: **Cabinet Nuss**
**10, rue Jacques Kablé**
**67080 Strasbourg Cedex (FR)**



(56) References cited:
- **TAKATSUGU WAKAHARA ET AL: "Fullerene/Cobalt Porphyrin Hybrid Nanosheets with Ambipolar Charge Transporting Characteristics", JOURNAL OF THE AMERICAN CHEMICAL SOCIETY, vol. 134, no. 17, 23 April 2012 (2012-04-23), pages 7204-7206, XP055502484, US ISSN: 0002-7863, DOI: 10.1021/ja211951v**
- **Konarev, D.M.; Neretin, I.S.; Slovokhotov, Y.L.; Yudanova, E.I.; Drichko, N.V.; Shul'ga, Y.M.; Tarasov, B.P.; Gumanov, L.L.;et al.: "New Molecular Complexes of Fullerenes C 60 and C 70 with Tetraphenylporphyrins [M(tpp)], in which M=H2, Mn, Co, Cu, Zn, and FeCl", Chemistry, A European Journal, 5 June 2001 (2001-06-05), pages 2605-2616, XP055502447, Retrieved from the Internet: URL:https://onlinelibrary.wiley.com/doi/ab s/10.1002/1521-3765%2820010618%297%3A12% 3C 2605%3A%3Aaid-chem26050%3E3.0.co%3B2-p ?jou rnalCode=15213765 [retrieved on 2018-08-27]**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



EP 3 635 796 B1

- ALEKSEY L. LITVINOV ET AL: "[60]Fullerene Complexes with Supramolecular Zinc Tetraphenylporphyrin Assemblies:? Synthesis, Crystal Structures, and Optical Properties", CRYSTAL GROWTH & DESIGN., vol. 5, no. 5, 11 August 2005 (2005-08-11) , pages 1807-1819, XP055502448, US ISSN: 1528-7483, DOI: 10.1021/cg050095m
- Andrey F Mironov: "Synthesis, Properties, and Potential Applications of Pohphyrin-Fullerenes", Macroheterocycles, 20 October 2011 (2011-10-20), pages 186-208, XP055502463, Moscow Retrieved from the Internet: URL:https://macroheterocycles.isuct.ru/en/node/248 [retrieved on 2018-08-27]

## Description

### FIELD OF THE INVENTION:

The present invention relates to a donor-acceptor single crystalline assembly. More particularly, the present invention relates to an assembly comprising the tetraphenylporphyrin and fullerene (C6o), process for preparation and use thereof in organic electronic devices.

### BACKGROUND AND PRIOR ART OF THE INVENTION:

Organic electronics has emerged as an active field of research promising energy efficient large-area lighting and light harvesting devices, flexible integrated circuits and displays. Recently, organic field effect transistors (OFETs) have been keenly monitored as a commercially viable technology. Since transistors are ubiquitous in our day-to-day life, OFETs are considered as low cost and efficient replacement for the present technologies. An important observation of electrical conductivity at the interfaces of two hetero molecules pointed towards ambipolar-OFETs and subsequently, lateral and bulk heterointerfaces of small molecules have been effectively utilized to compete with the high-performing counterparts. An ample way of making alternate (mixed) electron D-A stacks and thus enabled charge carrier mobility modulation has brought co-assembly of D/A small molecules as a potential candidate for bulk heterointerface formation. In recent years, a remarkable increase has been observed due to application of these assemblies in organic complementary-like circuits, organic light-emitting transistors, phototransistors, artificial skin and sensors.

In OFETs, since the first few nanometers thick layers of organic semiconductors at the dielectric- semiconductor interface contributes much to the total performance, charge carrier transport within the $\pi$-conjugated framework plays a remarkable role. Hence choice of D-A components, interface modification by using various electrodes, dielectric materials etc. contribute towards enhancement in the device performance. In this context, two-component D-A combinations leading to heterojunctions with molecular level order and precision is highly desired. The co assembly of a p-type and an n-type material has many advantages than that of the synthetically challenging single component ambipolar candidates and thereby got extensively tested in FETs. Although ambipolar charge transport materials are getting much attention, it remains as a challenging task to develop $\pi$-conjugated D-A assemblies that fulfill all the required parameters for a steady and balanced p- and n-type charge transport. In this direction, many research groups have focused on developing p/n junction assemblies with improved performance. However, ID p/n junction assembly with optimum charge transport features require judicious choice of donor, acceptor components and a promising strategy for coassembly formation.

Article titled "High-Mobility Field-effect transistors from large-area solution-grown aligned C60 single crystals" by H Li et al. published in J. Am. Chem. Soc., 2012, 134 (5), pp 2760- 2765 reports a solution processing method to grow large arrays of aligned $C_{60}$ single crystals. The disclosed well-aligned $C_{60}$ single-crystal needles and ribbons show electron mobility as high as 11 $cm^2V^{-1}s^{-1}$ (average mobility: 5.2 $\pm$ 2.1 $cm^2V^{-1}s^{-1}$ from needles; 3.0 $\pm$ 0.87 $cm^2V^{-1}s^{-1}$ from ribbons). This observed mobility is ~8-fold higher than the maximum reported mobility for solution-grown n-channel organic materials (1.5 $cm^2V^{-1}s^{-1}$) and is ~2-fold higher than the highest mobility of any n-channel organic material (~6 $cm^2V^{-1}s^{-1}$). Furthermore, the deposition method as disclosed is scalable to a 100 mm wafer substrate, with around 50% of the wafer surface covered by aligned crystals. Hence, the disclosed method facilitates the fabrication of large amounts of high-quality semiconductor crystals for fundamental studies, and with substantial improvement on the surface coverage of crystals, this method might be suitable for large-area applications based on single crystals of organic semiconductors.

Article titled "Large face to face tetraphenylporphyrin/fullerene nanoaggregates. A DFT study" by UJ Castillo et al. published in Organic Electronics; 2013, 14 (10), pp 2617-2627 reports large face to face tetraphenylporphyrin/fullerene nanoaggregates containing up six C60 units and six tetraphenylporphyrin (H2TPP) or tetraphenylporphyrinato-zinc (TPP-Zn) moieties. It has been found that most important contribution to the binding energy between fragments comes from dispersion interactions. The binding energies for Zn containing nanoaggregates are slightly higher than those for metal free ones what is not related to the difference in dispersion contributions to the binding energy but comes from DFT term. Center to center distances for large nanoaggregates are shorter than those for the complexes H2TPP/C60 and TPP-Zn/C60 and this effect is more obvious for metal free nanoaggregates.

Article titled" Enhancement of the open-circuit voltage and hole conduction of tetraphenylporphyrin /C60 multilayered photovoltaic device by the insertion of oxide hole collection layers" by E Itoh et al. published in Japanese Journal of Applied Physics, 2011, 50 Number 1S2 reports the photovoltaic properties of organic multilayered photovoltaic devices consisting of Indium-tin-oxide (ITO)/oxide/tetraphenylporphyrin ($H_2$TPP, ZnTPP)/fullerene ($C_{60}$)/bathocuproine (BCP)/A1 structures. The open-circuit voltage $V_{OC}$ increases with the thickness of porphyrin layers between 10 and 30 nm. The upper limit of $V_{OC}$ is attributed to the built-in potential and the energy difference between the highest occupied molecular orbital (HOMO) of $H_2$TPP and the lowest unoccupied molecular orbital (LUMO) of the $C_{60}$ layer $\Delta E$. The use of oxide hole collection layers, such as NiO and $MoO_3$, is effective for increasing the built-in potential across the organic

layers resulting in the improved $V_{OC}$. The resistance of the organic layers is dominated by the field-dependent bulk resistance of H2TPP films for V<VOC, whereas the kink above VOC was attributed to the relatively high Schottky barrier for holes at the ITO/H2TPP and ITO/MoO3 interfaces.

Article titled "Self-organization of porphyrins and fullerenes for molecular photoelectrochemical devices" by T Umeyama et al. published in Photosynthesis Research; 2006, 87 (1), pp 63-71 reports a variety of molecular assemblies of porphyrin as a donor and fullerene as an acceptor on electrodes for molecular photoelectrochemical devices. Highly efficient energy- and electron-transfer processes have been realized at gold electrodes modified with self-assembled monolayers of porphyrin- or fullerene linked systems mimicking light-harvesting and charge separation in bacterial photosynthesis. Highly ordered organization of porphyrins and fullerenes has also been achieved using step-by-step self-assembly of porphyrin and fullerene units by association with gold nanoparticles or dendrimers on tin oxide electrodes, which exhibit high power-conversion efficiency of up to 1.5%.

Article titled "Synthesis, electron transport, and charge storage properties of fullerene-zinc porphyrin hybrid nanodiscs" by A Garai et al. published in RSC Adv., 2014,4, 64119-64127 reports a donor (D)-acceptor (A) based system with the help of a newly synthesized Zn-porphyrin complex and $C_{60}$ fullerene. A series of spectroscopic methods, absorption spectroscopy (UV-Vis-NIR), powder X-ray diffraction (XRD), and X-ray photoelectron spectroscopy (XPS) measurements, confirm the existence of a charge transfer based complex between Zn-porphyrin and $C_{60}$ fullerene. By using this D-A based system, circular disc-like nano objects have been generated. With the help of these nano objects, a diode has been fabricated. Current-voltage (*I-V*) and the capacitance-voltage (*C-V*) analyses of the diode were performed. The critical role of the Ag-$C_{60}$-Zn-porphyrin and $C_{60}$-Zn-porphyrin-$SiO_x$ interfaces are clearly understood from the *I-V* characteristics of the present device.

Article titled "Fullerene/Sulfur-Bridged Annulene Cocrystals: Two-Dimensional Segregated Heterojunctions with Ambipolar Transport Properties and Photoresponsivity" by J Zhang et al. published in J. Am. Chem. Soc., 2013, 135 (2), pp 558-561 reports Fullerene/sulfur- bridged annulene cocrystals with a two-dimensional segregated alternating layer structure were prepared by a simple solution process. Single-crystal analysis revealed the existence of continuing $\pi$-$\pi$ interactions in both the donor and acceptor layers, which serve as transport paths for holes and electrons separately. The ambipolar transport behaviors were demonstrated with single-crystal field-effect transistors and rationalized by quantum calculations.

Article titled "Fullerene/Cobalt Porphyrin hybrid nanosheets with ambipolar charge transporting characteristics" by T Wakahara et al. published in J. Am. Chem. Soc., 2012, 134 (17), pp 7204-7206 reports a novel supramolecular nanoarchitecture, comprising $C_{60}$/Co porphyrin nanosheets prepared by a simple liquid-liquid interfacial precipitation method and fully characterized by means of optical microscopy, AFM, STEM, TEM, and XRD. They report that the highly crystalline $C_{60}$/Co porphyrin nanosheets have a simple (1:1) stoichiometry, and when incorporated in bottom-gate, bottom-contact field-effect transistors (FETs), they show ambipolar charge transport characteristics.

Article titled "New Molecular Complexes of Fullerenes C 60 and C 70 with Tetraphenylporphyrins [M(tpp)], in which M=H2, Mn, Co, Cu, Zn and FeCl" by D. Konarev et al. published in Chemistry, A European Journal Soc., 5 June 2001 (2001-06-05), pp 2605-2616 reports solvated complex with benzene in case of $H_2$TPP and with pyridine in case of ZnTPP. However, such publication strictly concerns solvated complex wherein the choice of the solvent is particularly crucial.

Article titled "[60]Fullerene Complexes with Supramolecular Zinc Tetraphenylporphyrin Assemblies, Synthesis, Crystal Structures, and Optical Properties" by Aleksey L. Litvinov et al. published in Crystal Growth & Design., Vol. 5, no. 5, (2005-08-11), pp 1807-1819 reports complexes where, ZnTPP is concaved by ligand coordination and then it forms complex with fullerene in a solvated form, so that these complexes involve solvent as well as ligands to obtain variety of packing motifs. However, this publication concerns bounds by different N- and O-containing ligands, which range from monomeric to pentameric such that its use cannot provide stable devices exhibited consistent mobilities even after months.

Article titled "Synthesis, Properties, and Potential Applications of Porphyrin-Fullerenes", by Andrey F. Mironov in Macroheterocycles 2011 4(3) 186-208 overviews the most popular methods to prepare porphyrin complexes with C60 fullerene exhibiting various types of linkage is given with the main focus on the Prato reaction and its modifications. The discussion of porphyrin-fullerene complexes will include the structures of noncovalently linked porphyrin-fullerenes along with the covalently linked complexes; the chemistry and design of supramolecular ensembles will be covered rather comprehensively.

One-dimensional (ID) nanostructures of $\pi$-conjugated molecules exhibiting excellent charge carrier mobilities have found much interest in organic electronic devices. Even though it is tedious to form such structures, the availability of highly delocalized electron and hole carriers in these donor (D)-acceptor (A) co-assemblies realize ambipolar charge transport. The major drawback of prior art assembly is that the ID p/n junction assembly with optimum charge transport features require judicious choice of donor, acceptor components and a promising strategy for coassembly formation.

Therefore, there is need to develop 1D p/n junction assembly with optimum charge transport features. Accordingly, the present invention provides a composition comprising the tetraphenylporphyrin and fullerene ($C_{60}$) which pro-

vides improved ambipolar charge transport properties for OFETs.

## OBJECTS OF THE INVENTION:

The main objective of the present invention is to provide an assembly comprising the tetraphenylporphyrin and fullerene ($C_{60}$).

Another objective of the present invention is to provide a process for the preparation of assembly of tetraphenylporphyrin and fullerene ($C_{60}$).

Yet another objective of the present invention is to provide an organic Field-Effect Transistor (OFET) comprising assembly of tetraphenylporphyrin and fullerene ($C_{60}$).

## SUMMARY OF THE INVENTION:

The present invention provides an assembly consisting of the tetraphenylporphyrin and $C_{60}$ fullerene for use in organic filed effect transistor according to main claim 1.

In an embodiment, the present invention provides a process for the preparation of assembly of tetraphenylporphyrin and $C_{60}$ fullerene according to the invention, this process comprising the steps of:

a) mixing the tetraphenylporphyrin in *m*-xylene and $C_{60}$ in *m*-xylene followed by allowing the resulting solution to stand over 2 to 5 min without disturbance;

b) adding two drops 10μL solution of step (a) on silicon substrates followed by drying for the period in the range of 2 to 4 hr to afford assembly of tetraphenylporphyrin and $C_{60}$ fullerene.

In another embodiment, the present invention provides an organic field-effect transistor comprising assembly of tetraphenylporphyrin and $C_{60}$ fullerene according to main claim 2.

Additional embodiments of the organic field-effect transistor of the present invention are detailed in the set of claims e.g. in claims 3-7.

## BRIEF DESCRIPTION OF THE DRAWINGS:

**Figure 1:** a) SEM and b) TEM images of the PIN junction assembly of ZnTPP-$C_{60}$. c) High-resolution TEM image of ZnTPP-$C_{60}$ cocrystals with d) selected area electron diffraction pattern. Elemental mapping of the ZnTPP-$C_{60}$ cocrystals for e) carbon, f) nitrogen and g) zinc, showing an even distribution of ZnTPP.

**Figure 2:** a) Comparison of Raman spectrum of the individual components and ZnTPP-$C_{60}$ cocrystals, peaks corresponding to ZnTPP and $C_{60}$ are marked by green circles and red squares, respectively, b) UV-Vis absorption spectra of porphyrins $H_2$TPP, ZnTPP and corresponding cocrystals $H_2$TPP-$C_{60}$, ZnTPP-$C_{60}$-XP spectra of ZnTPP and ZnTPP-$C_{60}$ cocrystals showing variations of c) pyrrolic N 1*s*and d) Zn $P_{3/2}$ region of the spectrum.

**Figure 3:** a) XP spectra of $H_2$TPP and $H_2$TPP-$C_{60}$ cocrystals showing variations of pyrrolic and iminic N 1*s* region of the spectrum, b) OM images of OFET device showing $H_2$TPP-$C_{60}$ cocrystals covering the channel.

**Figure 4:** a) Schematic of the FET device structure.FET Characteristics of ZnTPP-$C_{60}$ p-n junction assemblies, typical b) transfer and d) output characteristics in n-channel operation mode under positive drain bias. Typical c) transfer and e) output characteristics in p-channel operation mode under negative drain bias.

**Figure 5:** OM images of ZnTPP-$C_{60}$ cocrystals from m-xylene.

**Figure 6:** SEM images of ZnTPP-$C_{60}$ cocrystals from m-xylene.

**Figure 7:** a)-c) TEM images of ZnTPP-$C_{60}$ cocrystals from *m*-xylene, showing d) lattice planes with *d*-spacing's of 1.231 and 0.898 nm

**Figure 8:** OM images of $H_2$TPP-$C_{60}$ cocrystals from m-xylene.

**Figure 9:** SEM images of $H_2$TPP-$C_{60}$ cocrystals from m-xylene.

**Figure 10:** a)-c) TEM image of $H_2$TPP-$C_{60}$ cocrystals from m-xylene, showing d), e) lattice planes with d-spacing of 1.217 nm and f) SAED pattern indicating single crystalline ordering of the cocrystals.

**Figure 11:** a) TEM image of $H_2$TPP-$C_{60}$ cocrystals from m-xylene with elemental mapping for b) carbon and c) nitrogen.

**Figure 12:** FT-IR spectra of $H_2$TPP-$C_{60}$, ZnTPP-$C_{60}$ cocrystals.

**Figure 13:** Comparison of Raman Spectra of $C_{60}$, $H_2$TPP, $H_2$TPP-$C_{60}$ cocrystals, peaks corresponding to $H_2$TPP and $C_{60}$ are marked by green circles and red squares, respectively

**Figure 14:** UV-Vis spectra of toluene solution of $H_2$TPP-$C_{60}$ and ZnTPP-$C_{60}$ cocrystals.

**Figure 15:** Comparison of TGA of a) $C_{60}$, ZnTPP, ZnTPP-C6o cocrystals and b) $C_{60}$, $H_2$TPP, $H_2$TPP-$C_{60}$ cocrystals.

**Figure 16:** Comparison of Maldi of a) $C_{60}$, ZnTPP, ZnTPP-C6o cocrystals and b) $C_{60}$, $H_2$TPP, $H_2$TPP-$C_{60}$ cocrystals.

**Figure 17:** Comparison of the HOMO-LUMO levels of $H_2$TPP, ZnTPP and $C_{60}$.

**Figure 18:** FET characteristics of $H_2$TPP-$C_{60}$ single crystalline p-n junctions. Typical a) transfer and b) output characteristics in p-channel operation mode under negative drain bias. Typical c) transfer and d) output characteristics in n-channel operation mode under positive drain bias.

**Figure 19:** Plot showing the leakage current of a) ZnTPP-$C_{60}$ and b) $H_2$TPP-$C_{60}$ OFET device.

## DETAILED DESCRIPTION OF THE INVENTION

The invention will now be described in detail in connection with certain preferred and optional embodiments, so that various aspects thereof may be more fully understood and appreciated.

In the view of above, the present invention provides an assembly comprising the tetraphenylporphyrin and fullerene ($C_{60}$), process for preparation thereof and use thereof in organic electronic devices.

In an embodiment, the present invention provides an assembly comprising the tetraphenylporphyrin and fullerene ($C_{60}$).

The assembly may additionally comprises transition metal ion selected from Zinc (Zn), Co (Cobalt), Copper (Cu) or Nickel (Ni). The assembly is made up of $H_2$TPP and fullerene or ZnTPP and fullerene and is in the form of crystalline rods, nanowires, nanofibers, nanobelts or nanosheets.

In one embodiment, the assembly is used in organic field-effect transistor (OFET) wherein tetraphenylporphyrin is selected from $H_2$TPP or ZnTPP.

In an embodiment, the present invention provides a process for the preparation of assembly of tetraphenylporphyrin and $C_{60}$ fullerene comprising the steps of:

a) mixing the tetraphenylporphyrin in *m*-xylene and $C_{60}$ in *m*-xylene followed by allowing the resulting solution to stand over 2 to 5 min without disturbance;

b) adding two drops 10μL solution of step (a) on silicon substrates followed by drying for the period in the range of 2 to 4 hr to afford assembly of tetraphenylporphyrin and $C_{60}$ fullerene.

The tetraphenylporphyrin is selected from $H_2$TPP or ZnTPP. The m-xylene is the selected solvent.

The ID assemblies of 5,10,15,20-tetraphenylporphyrins ($H_2$TPP, ZnTPP) and fullerene ($C_{60}$) exhibit high ambipolar mobility in the range of 0.8 to 3.4 $cm^2$/Vs for electrons and holes with high ON/OFF ratio and low threshold voltage. The Chemical structure of tetraphenylporphyrins $H_2$TPP, ZnTPP and fullerene ($C_{60}$) is shown in scheme 1.

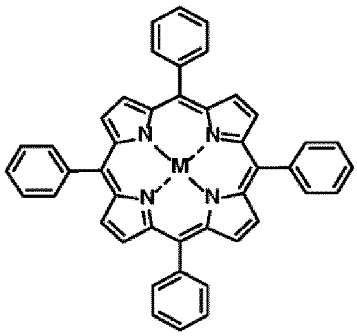

$H_2$TPP: M = $H_2$

ZnTPP: M = Zn

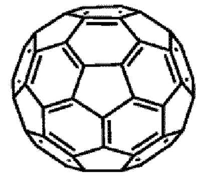

$C_{60}$

Scheme 1: Chemical structure of tetraphenylporphyrins $H_2$TPP, ZnTPP and fullerene ($C_{60}$) used for ambipolar OFETs.

The crystalline rods with high aspect ratio by drop casting *m*-xylene solution of $H_2$TPP/ZnTPP and $C_{60}$ (1: 1) on various substrates, preferably on Silicon wafer are prepared. The evaporation of solvent at room temperature assisted the formation of uniform rods on the surface within 2 hours. Attempts to prepare the assembly using $CS_2$, chlorobenzene, dichlorobenzene solutions of porphyrins and $C_{60}$ (1: 1) resulted in the formation of ill-defined aggregates along with rods. Optical microscope (OM) (Figure 5), scanning electron microscope (SEM) (Figure 1a, 6) and transmission electron microscope (TEM) images (Figure 1b, 7) show that the rods are formed uniformly with good aspect ratio. Each crystalline rod has a length of several tens of micrometers and thickness of a few micrometers. High magnification image showed the presence of two-dimensional lattice planes with *d*-spacing of 1.23 and 0.90 nm (Figure 1c, 7). Selected area electron diffraction pattern confirmed single crystallinity for the rods (Figure 1d). Additionally, the even distribution of porphyrins in the crystalline rods are confirmed by elemental mapping for carbon (Figure 1e), nitrogen (Figure 1f) and zinc (Figure 1g). Similarly, $H_2$TPP-$C_{60}$ cocrystals are also prepared and characterized (Figure 8-13).

Comparison of solid state Fourier transform infrared (Figure 14) and Raman spectra (Figure 15) of individual components with crystalline rods confirmed the presence of both porphyrin and $C_{60}$ in the cocrystal. In addition, cocrystals formed are found to be different from those of the individual component assemblies and UV-visible spectra of cocrystals dissolved in toluene showed characteristic features of both porphyrin and $C_{60}$ (Figure 14). However, solid state UV-Vis spectra indicated significant changes of the corresponding peaks due to the ground state D-A CT interactions (Figure 2b). This is clearly observed in the case of ZnTPP- $C_{60}$ as a long wavelength tail in the UV-Vis spectrum of the coassembly (Figure 2b, blue solid line). X-ray photoelectron spectroscopy (XPS) has been used to measure changes in the electron density distribution of hetero-atoms of the donors upon cocrystal formation with $C_{60}$ (Figure 2c,d and 3a). XP spectrum of the chemically equivalent ZnTPP nitrogen atoms give a single peak corresponding to N 1*s* with a binding energy of 398.6 eV (Figure 2c). Similarly, $Zn^{2+}$ in ZnTPP exhibits signal at the Zn $2P_{3/2}$ region of the XP spectrum with binding energy of 1022.4 eV (Figure 2d). However, in the case of ZnTPP-$C_{60}$ cocrystal, XP spectrum shows a positive shift for both N 1*s* (1.4 eV) and Zn $2P_{3/2}$ (1.51 eV) peaks. The observed positive shift could be attributed to the enhanced CT interaction between ZnTPP and $C_{60}$ in the cocrystal. In the case of $H_2$TPP-$C_{60}$, two peaks are present for nitrogen atoms with binding energy of 398.3 eV (iminic (=N-) and 400.3 eV (pyrrolic (-NH-)) (Figure 3a). A positive shift for N 1*s* with 1.2 and 1.3 eV for pyrrolic N and iminic N, respectively is observed upon complexation with $C_{60}$. Thermogravimetric analysis (Figure 15) also confirmed the formation of co-crystalline assemblies. The above series of experiments reveal the formation of cocrystal assemblies and even distribution of the individual components throughout the assembly. However, a noticeable difference in the long wavelength region of ZnTPP-$C_{60}$ cocrystal UV-Vis spectrum motivated us to do further investigation on it.

In another embodiment, the present invention provides an organic field-effect transistor (OFET) comprising assembly of tetraphenylporphyrin and fullerene ($C_{60}$).

In preferred embodiment, the organic field-effect transistor (OFET) shows high ambipolar mobility in the range of 0.8 to 3.4 $cm^2$/Vs.

In another preferred embodiment, the organic field-effect transistor (OFET) shows electron mobility for ZnTPP-C60is 1.35 $cm^2$/V/s and electron mobility for $H_2$TPP-$C_{60}$ is 0.86 $cm^2$/V/s.

In yet another preferred embodiment, the organic field-effect transistor (OFET) shows hole mobility for ZnTPP-$C_{60}$ is 3.42 $cm^2$/V/s and hole mobility for $H_2$TPP-$C_{60}$ is of 1.12 $cm^2$/V/s.

The single crystals of the tetraphenylporphyrins and $C_{60}$ coassembly (1:1) obtained by slow evaporation of toluene solutions exhibited a zigzag chain of alternating porphyrin/$C_{60}$ arrays. The ease of coassembly formation, though the area of contact is very less in a binary assembly of a planar (porphyrin) and spherical ($C_{60}$) structures, is due to the attractive interaction between the electron deficient centre of porphyrin or metalloporphyrin and the olefinic bonds of fullerenes.

The single crystalline p-n junction rods of $H_2$/ZnTPP-$C_{60}$ are tested for the ambipolar charge carrier mobility in transistor device. After several trials with various combinations of electrodes and dielectrics, top-contact/bottom gate transistors of structure, glass/$Al_2O_3$/PVA/organic active material/Au (channel length 50 $\mu$m, width 1 mm) are fabricated for ambipolar measurement (Figure 3b,4a). Figure 4b and 4c shows typical transfer characteristics of the OFET devices having V-shaped curves. The output characteristics in n- channel (Figure 4d) and p-channel (Figure 4e) operation modes exhibit excellent gate modulation. The electron and hole mobility values of ZnTPP-$C_{60}$ rods are found to be 1.6 and 3.9 $cm^2$/V/s, with ON/OFF ratios of $1 \times 10^4$ and $0.5 \times 10^3$, respectively. A comparison of the nature of electronic excitations in D-A combination of tetraphenylporphyrins and $C_{60}$ suggests that ZnTPP-$C_{60}$ exhibit strong CT nature than $H_2$TPP-$C_{60}$. Hence $C_{60}$ carbon atom at the junction of two fused six-member rings is placed on the top of porphyrins keeping a closest distance between them. Such supportive supramolecular interactions between the planar porphyrin and the spherical fullerene surfaces control the topology of these systems in the ground and electronically excited states. It is observed that, the presence of metal ion allows better orbital overlap between $C_{60}$ and ZnTPP fragments (Figure 16) facilitating polaron derealization along the aggregate to enhance the mobility values.

The H2TPP and C6o rods exhibited a balanced hole and electron mobility values of 0.81 and 1.02 $cm^2$/V/s, with ON/OFF ratios of $10^4$ and $10^3$, respectively (Figure 17). An increase in the threshold voltage ($V_{th}$) in p-region is observed for both $H_2$TPP and ZnTPP co-assemblies and that could be attributed to the contact resistance resulting from the no ideal interface (Table 1). Figure 18 represents the leakage current variation with respect to the gate voltage and a very low leakage through the gate dielectric, in the order of $10^{-9}$ A, confirms that no significant influence of leakage on device output characteristics.

**Table 1:** Summary of device characteristics

| Microstructure used for OFET fabrication | $V_{th}$ (V) | | $\mu$ ($cm^2$/Vs) | | $I_{on}$ / $I_{off}$ Ratio | $I_{on}$ / $I_{off}$ Ratio |
|---|---|---|---|---|---|---|
| | n-type | p-type | electron | hole | n-type | p-type |
| $H_2$TPP-$C_{60}$ Cocrystals | 0.76 | 5.66 | 0.86 | 1.12 | $10^4$ | $10^3$ |
| ZnTPP-$C_{60}$ Cocrystals | 0.44 | 3.25 | 1.35 | 3.42 | $10^4$ | $0.5 \times 10^3$ |

These devices are stable and exhibited consistent mobilities even after months. A rational choice of the Al as gate and Au as source, drain electrodes and a suitable combination of PVA-$Al_2O_3$ as dielectric along with high quality single crystals resulted in the high mobility values. An imbalanced electron and hole mobilities of ZnTPP-$C_{60}$ rods might be attributed to the slight mismatch between the work function of the Al electrode and the HOMO and/or LUMO level of the ambipolar semiconductor layer. Moreover, the presence of more traps for electrons than for holes in the semiconductor and the semiconductor/dielectric interfaces also contribute towards lower electron mobility.

In still another embodiment, the assembly is used in many electronic devices such organic field effect transistor, organic light-emitting transistors, phototransistors or sensors.

The invention will now be described in detail in connection with certain preferred and optional embodiments, so that various aspects thereof may be more fully understood and appreciated.

Examples Following examples are given by way of illustration therefore should not be construed to limit the scope of the invention.

**Example 1: Synthesis of $H_2$TPP:**

Freshly distilled pyrrole (56 ml, 0.8 mole) and 80 ml(0.8 mole) of reagent grade benzaldehyde are added to RB contains refluxing reagent grade propionic acid. Reflux it for 30 min, the solution is allow cooling and filtered, and the filter compound is washed thoroughly with methanol. After a hot water wash, the resulting purple crystals are air dried, and finally dried in vacuo to remove adsorbed acid to yield 25 g (20%, yield) of TPP.

**Example 2: Synthesis of ZnTPP:**

The purple crystals $H_2$TPP has taken in RB contains DCM to this add $Zn(OAc)_2$-$2H_2O$ stir the reaction mixture at room temperature overnight. The color of the solution changed from dark-purple to reddish-purple during the course

of the reaction. The reaction mixture was purified by column chromatography (silica gel, DCM) and the purple band was collected. The solvent was removed under reduced pressure to yield ZnTPP as a purple solid (77%).

**Example 3: $H_2$TPP/ZnTPP-Fullerene ($C_{60}$) Assembly:**

0.5 ml of equimolar m-xylene solutions of $H_2$TPP/ZnTPP and $C_{60}$ were simultaneously mixed at ambient condition. The resulting solution was allowed to stand over 2 min without disturbance. 2 drops (10 μE) of a $H_2$TPP/ZnTPP-$C_{60}$ mixed m-xylene solution was placed on silicon substrates and dried over 2 hr. The formation of rod shaped assemblies were observed after 2 hr.

**Example 4: Characterization of H2TPP/ZnTPP-Fullerene ($C_{60}$) Assembly:**

**a) PXRD, Raman, and Solid UV-Vis Absorption**

0.5 ml of equimolar m-xylene solutions of $H_2$TPP/ZnTPP and $C_{60}$ were simultaneously mixed at ambient condition. The resulting solution was allowed to stand over a week without disturbance (sample is covered with Al foil). A brown coloured crystals were formed at bottom of the glass vial.

**b) Optical Microscope (OM), Scanning Electron Microscope (SEM), Atomic Force Microscope (AFM)**

The OM, ESEM and AFM samples were prepared by drop casting (10 μL) of the mixed solution of $H_2$TPP/ZnTPP-$C_{60}$ in *m*-xylene on silicon substrates and dried over 2 hr. After complete evaporation of the solvent, samples were directly used for OM and AFM analyses. The samples were coated with Au in an ion coater for 5 min before ESEM analysis.

**c) Transmission Electron Microscope (TEM)**

The samples for TEM were prepared by drop casting the mixed solutions of $H_2$TPP/ZnTPP-$C_{60}$ in *m*-xylene on carbon coated Cu grid and allowed to dry overnight in desiccators.

**Example 5: OFET Fabrication:**

Bottom gate top-contact OFET device geometry has been followed to fabricate microstructure based device fabrication. The device consists of Aluminum (Al) bottom gate, $Al_2O_3$/PVA bi-layer dielectric system, organic active channel and Gold (Au) source-drain electrodes. Initially Al gate contact of thickness -300 nm was deposited on pre-cleaned glass substrate. A part of Al film was then anodized to form $Al_2O_3$ layer to serve as a gate dielectric layer. Due to anodization, the surface becomes rough and it causes poor capacitive coupling. In order to achieve a smooth surface and to have organic interface, the $Al_2O_3$ surfaces is spincoated with 30 nm thick PVA (MW~ 125,000) layer of 5 wt%. In order to remove unwanted residuals, the PVA coated substrate was further annealed at 100 °C for 30 min. The main intension in providing bi-layer dielectric system is to minimize the interface induced effects and to establish an organic interface. After successful fabrication of bilayer dielectric system, self-assembled organic microstructures have been deposited on it. Finally, 120 nm thick Au source-drain electrodes were thermally grown through shadow a mask. The capacitance value used for mobility calculation was obtained from CV measurements.

**OFET Characterization:**

Transistors were characterized with the help of Keithley 4200 semiconductor characterization system, which is assembled with a homemade probe-station of three probes. While collecting current-voltage characteristics, the devices were placed in chamber under Argon (Ar) atmosphere. By keeping fixed source-drain bias, while sweeping gate voltage with constant sweep rate, device transfer characteristics have been measured. Similarly, the output characteristics were measured by sweeping drain voltage for fixed gate bias.
The following formula has been used to calculate the mobility values.

$$I_D = \frac{W}{L} \mu C_i (V_G - V_{th}) V_D$$

Wherein,
$I_D$: Drain Current; W: Channel Width; L: Channel Length; μ: Carrier Mobility; $C_i$: Capacitance; $V_G$: Gate Voltage; $V_{th}$:

Threshold Voltage; $V_D$ = Drain Voltage.

The measured capacitance of the dielectric system used in the OFET of present invention is in the range of 10 to 15 $nF/cm^2$.

**Advantages of the invention:**

1. A common use of the FET is as an amplifier.
2. FET acts as a chemical sensor.
3. FET acts as a Biosensor.
4. In organic light emitting diodes (OLEDs), photovoltaic cells,
5. The OFET prepared by using assembly of present invention shows very high ambipolar mobility.

## Claims

1. An assembly consisting of the tetraphenylporphyrin and $C_{60}$ fullerene for use in organic field-effect transistor, wherein said tetraphenylporphyrin is selected from $H_2TPP$ or ZnTPP, wherein said assembly is made up of $H_2TPP$ and fullerene or ZnTPP and fullerene and is in the form of crystalline rods, nanowires, nanofibers, nanobelts, and nano sheets.

2. A process for the preparation of an assembly of tetraphenylporphyrin and $C_{60}$ fullerene as claimed in claim 1, wherein said process comprising the steps of:

   a) mixing the tetraphenylporphyrin in *m*-xylene and $C_{60}$ in *m*-xylene followed by allowing the resulting solution to stand over 2 to 5 min without disturbance;
   b) adding two drops 10μL solution of step (a) on silicon substrates followed by drying for a period in the range of 2 to 4 hr to afford assembly of tetraphenylporphyrin and $C_{60}$ fullerene.

3. An organic field-effect transistor comprising an assembly consisting of tetraphenylporphyrin and $C_{60}$ fullerene, wherein said tetraphenylporphyrin is selected from $H_2TPP$ or ZnTPP.

4. The organic field-effect transistor as claimed in claim 4, wherein said organic field-effect transistor further comprises of Aluminium as a bottom gate, $A1_2O_3$/PVA as a bi-layer dielectric system and Gold as a source-drain electrodes.

5. The organic field-effect transistor as claimed in claim 4, wherein said organic field-effect transistor shows high ambipolar mobility in the range of 0.8 to 3.4 $cm^2/V/s$.

6. The organic field-effect transistor as claimed in claim 4, wherein said organic field-effect transistor shows electron mobility in the range of 0.86 cm /V/s to 1.12 $cm^2/V/s$.

7. The organic field-effect transistor as claimed in claim 4, wherein said organic field-effect transistor shows hole mobility in the range of 1.35 cm /V/s to 3.42 $cm^2/V/s$.

## Patentansprüche

1. Aggregat, bestehend aus Tetraphenylporphyrin und $C_{60}$-Fulleren, zur Verwendung in einem organischen Feldeffekttransistor, wobei das Tetraphenylporphyrin ausgewählt ist aus $H_2TPP$ und ZnTPP, wobei das Aggregat aus $H_2TPP$ und Fulleren oder ZnTPP und Fulleren gebildet ist und in der Form von kristallinen Stäbchen, Nanodrähten, Nanofasern, Nanobändern und Nanoblättern vorliegt.

2. Verfahren zur Herstellung eines Aggregats von Tetraphenylporphyrin und $C_{60}$-Fulleren gemäß Anspruch 1, wobei das Verfahren die Schritte umfasst:

   a) Mischen des Tetrahydroporphyrins in *m*-Xylen und $C_{60}$ in *m*-Xylen, gefolgt von 2 bis 5 min Ruhen lassen der erhaltenen Lösung ohne Störung;

b) Zugeben von zwei Tropfen von 10 μl Lösung von Schritt (a) auf Siliciumsubstrate, gefolgt von Trocknen für einen Zeitraum in dem Bereich von 2 bis 4 h, um Aggregieren von Tetraphenylporphyrin und $C_{60}$-Fulleren zu erhalten.

**3.** Organischer Feldeffekttransistor, umfassend ein Aggregat bestehend aus Tetraphenylporphyrin und $C_{60}$-Fulleren, wobei das Tetraphenylporphyrin ausgewählt ist aus $H_2$TPP und ZnTPP.

**4.** Organischer Feldeffekttransistor gemäß Anspruch 4, wobei der organische Feldeffekttransistor ferner Aluminium als ein unteres Gate, $Al_2O_3$/PVA als ein Doppelschicht-Dielektrikumsystem und Gold als eine Source-Drain-Elektrode umfasst.

**5.** Organischer Feldeffekttransistor gemäß Anspruch 4, wobei der organische Feldeffekttransistor hohe ambipolare Mobilität in dem Bereich von 0,8 bis 3,4 $cm^2$/V/s aufweist.

**6.** Organischer Feldeffekttransistor gemäß Anspruch 4, wobei der organische Feldeffekttransistor Elektronenmobilität in dem Bereich von 0,86 cm/V/s bis 1,12 $cm^2$/V/s aufweist.

**7.** Organischer Feldeffekttransistor gemäß Anspruch 4, wobei der organische Feldeffekttransistor Löchermobilität in dem Bereich von 1,35 cm/V/s bis 3,42 $cm^2$/V/s aufweist.

## Revendications

**1.** Assemblage constitué de la tétraphénylporphyrine et de fullerène $C_{60}$ pour une utilisation dans un transistor organique à effet de champ, ladite tétraphénylporphyrine étant choisie parmi $H_2$TPP ou ZnTPP, ledit assemblage étant composé de $H_2$TPP et de fullerène ou de ZnTPP et de fullerène et étant sous la forme de bâtons cristallins, de nanofils, de nanofibres, de nanocourroies et de nanofeuilles.

**2.** Procédé pour la préparation d'un assemblage de tétraphénylporphyrine et de fullerène $C_{60}$ selon la revendication 1, ledit procédé comprenant les étapes de :

a) mélange de la tétraphénylporphyrine dans le *m*-xylène et de $C_{60}$ dans le *m*-xylène suivi par le fait de laisser la solution résultante reposer pendant 2 à 5 min sans perturbation ;
b) ajout de deux gouttes de 10 μL de solution de l'étape (a) sur des substrats de silicium suivi par un séchage pendant une période dans la plage de 2 à 4 heures pour donner un assemblage de tétraphénylporphyrine et de fullerène $C_{60}$.

**3.** Transistor organique à effet de champ comprenant un assemblage constitué de tétraphénylporphyrine et de fullerène $C_{60}$, ladite tétraphénylporphyrine étant choisie parmi $H_2$TPP ou ZnTPP.

**4.** Transistor organique à effet de champ selon la revendication 4, ledit transistor organique à effet de champ comprenant de l'aluminium en tant que grille inférieure, $Al_2O_3$/PVA en tant que système diélectrique bicouche et de l'or en tant qu'électrodes source-drain.

**5.** Transistor organique à effet de champ selon la revendication 4, ledit transistor organique à effet de champ présentant une mobilité ambipolaire élevée dans la plage de 0,8 à 3,4 $cm^2$/V/s.

**6.** Transistor organique à effet de champ selon la revendication 4, ledit transistor organique à effet de champ présentant une mobilité d'électrons dans la plage de 0,86 $cm^2$/V/s à 1,12 $cm^2$/V/s.

**7.** Transistor organique à effet de champ selon la revendication 4, ledit transistor organique à effet de champ présentant une mobilité de trous dans la plage de 1,35 $cm^2$/V/s à 3,42 $cm^2$/V/s.

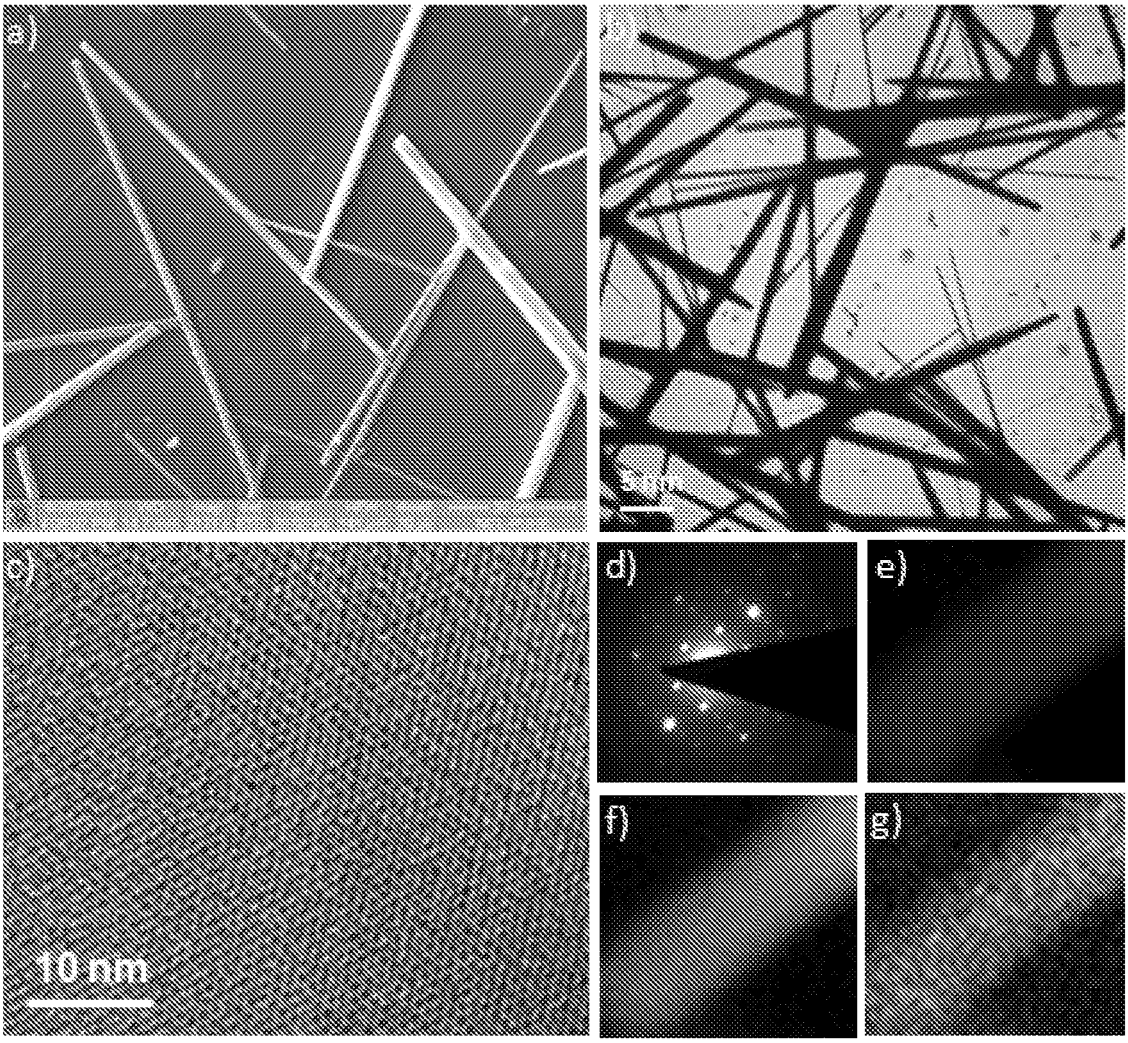


Fig. 1

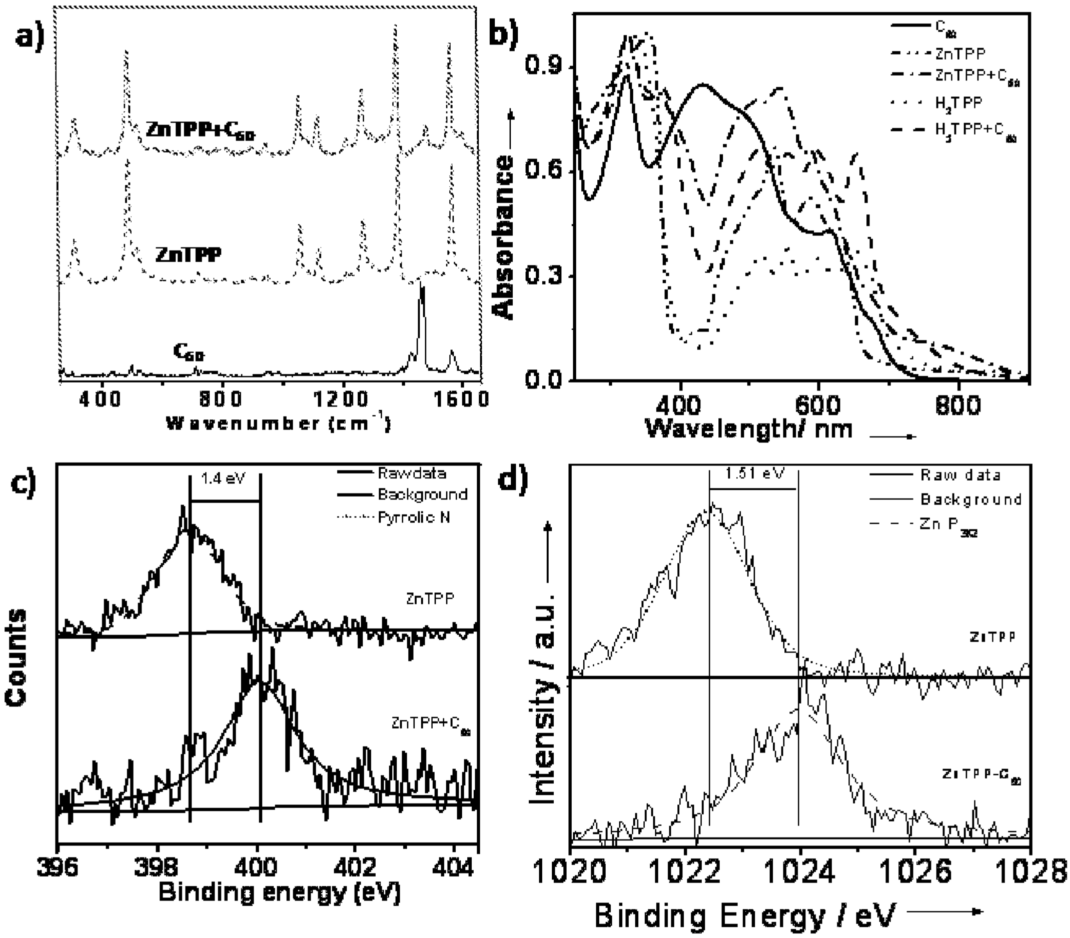
a)
ZnTPP+C60
ZnTPP
C60
Wavenumber (cm-1)
b)
C60
ZnTPP
ZnTPP+C60
H2TPP
H2TPP+C60
Absorbance
Wavelength/ nm
c)
1.4 eV
Rawdata
Background
Pyrrolic N
ZnTPP
ZnTPP+C60
Counts
Binding energy (eV)
d)
1.51 eV
Raw data
Background
Zn P3/2
ZnTPP
ZnTPP-C60
Intensity / a.u.
Binding Energy / eV

Fig. 2

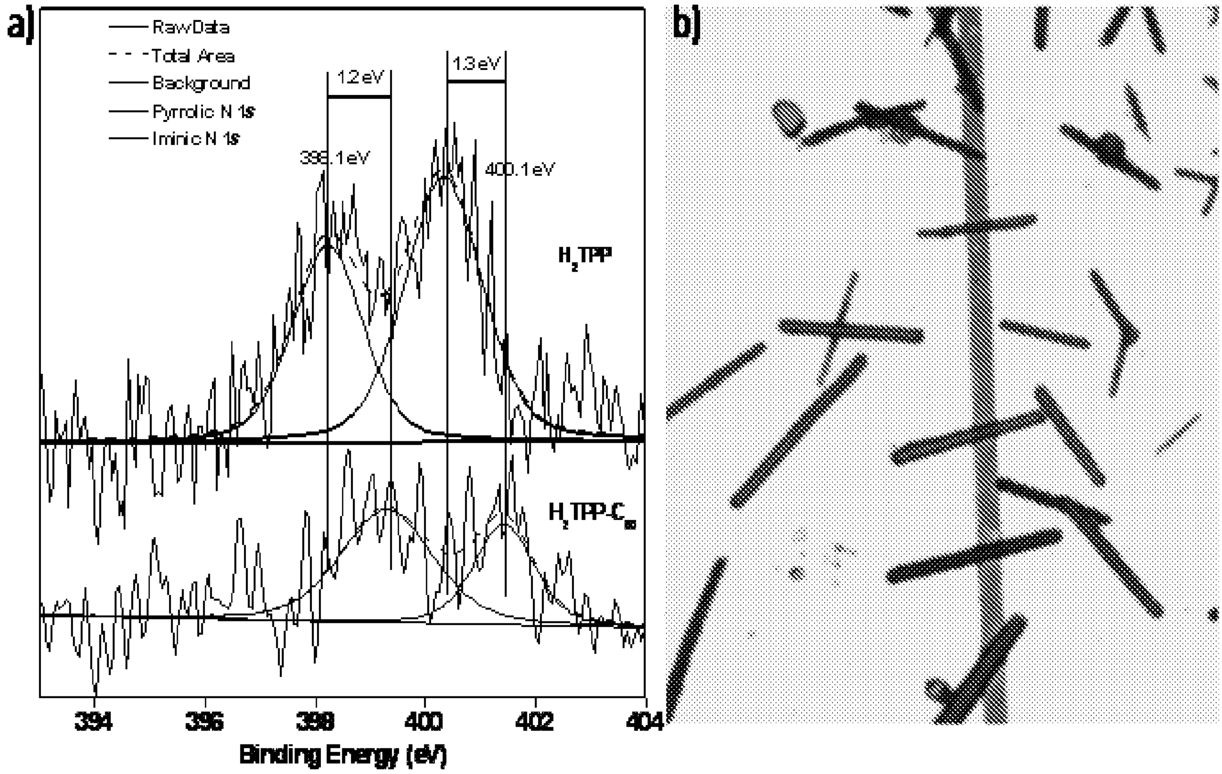
a)
Raw Data
Total Area
Background
Pyrrolic N 1s
Iminic N 1s
1.2 eV
1.3 eV
400.1 eV
H2TPP
H2TPP-C60
394
396
398
400
402
404
Binding Energy (eV)
b)

Fig. 3

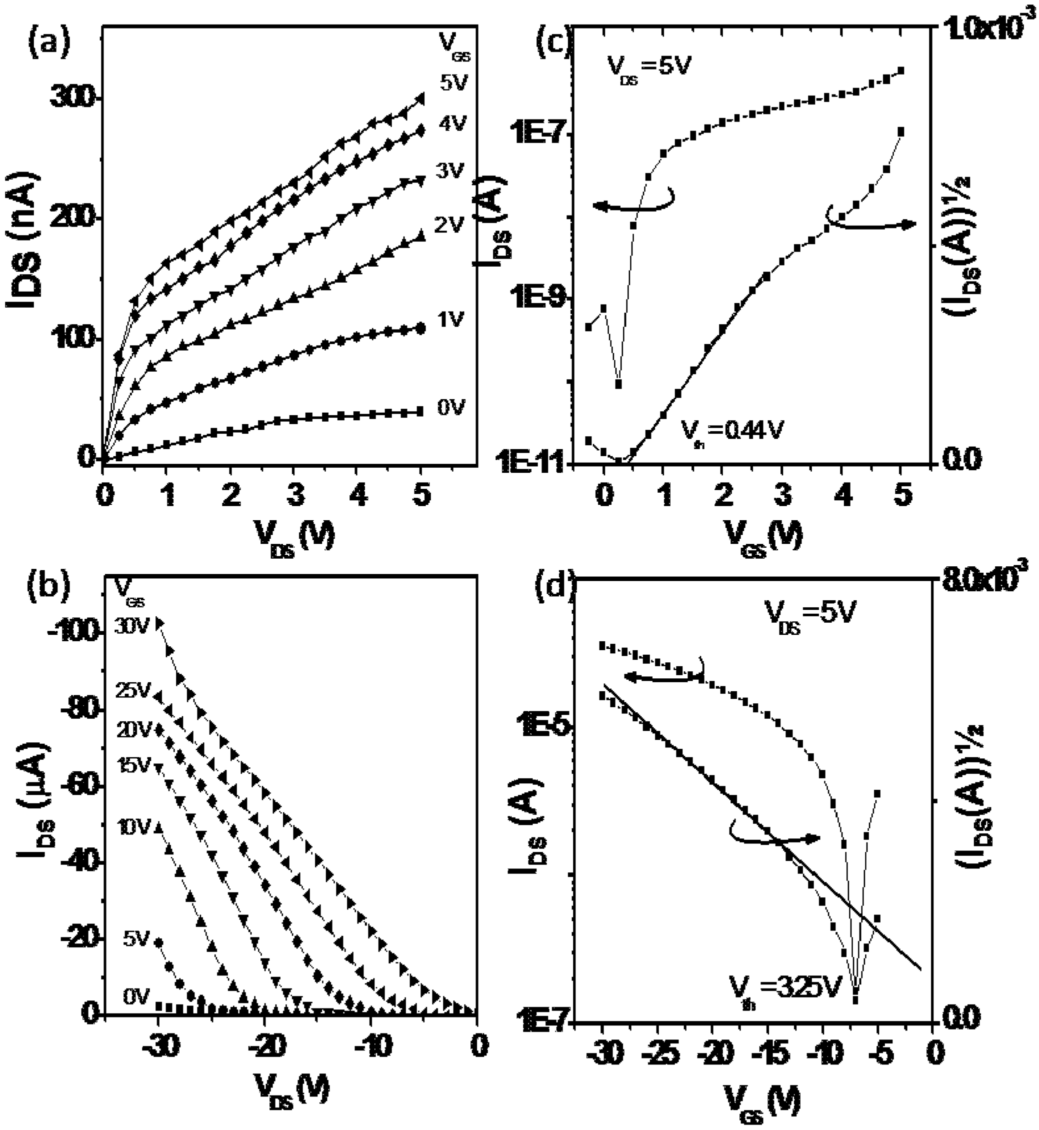
(a)
$V_{GS}$
5V
4V
3V
2V
1V
0V
$I_{DS}$ (nA)
$V_{DS}$ (V)
(b)
$V_{GS}$
30V
25V
20V
15V
10V
5V
0V
$I_{DS}$ (μA)
$V_{DS}$ (V)
(c)
$V_{DS}$=5V
$V_{th}$=0.44V
$I_{DS}$ (A)
$(I_{DS}(A))^{1/2}$
$V_{GS}$ (V)
(d)
$V_{DS}$=5V
$V_{th}$=3.25V
$I_{DS}$ (A)
$(I_{DS}(A))^{1/2}$
$V_{GS}$ (V)

Fig. 4

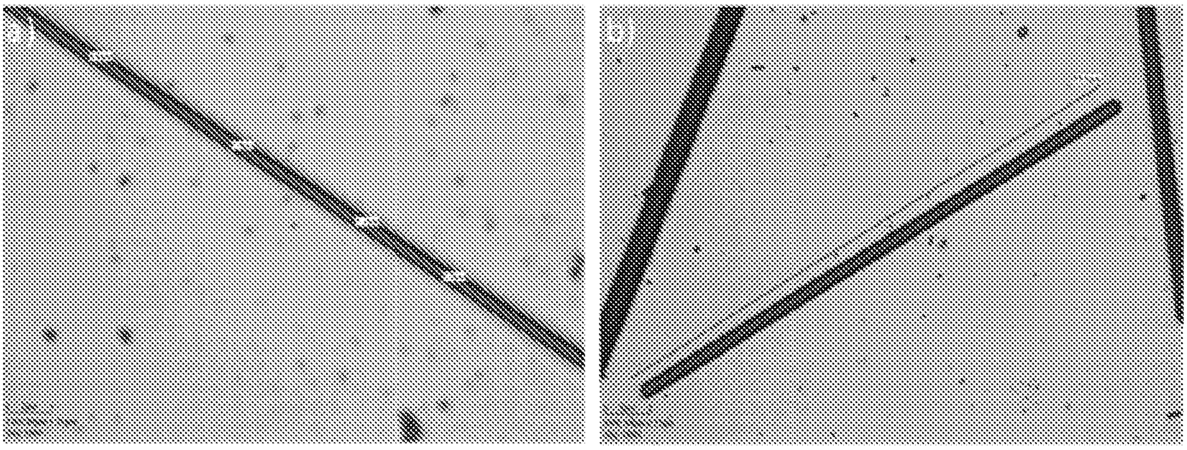


Fig. 5

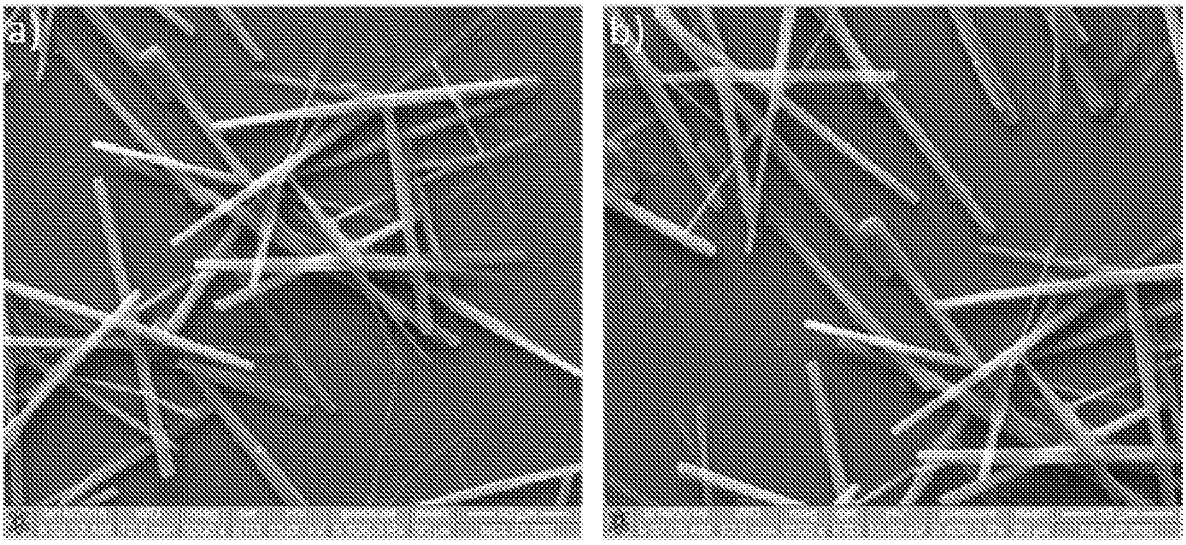


Fig. 6

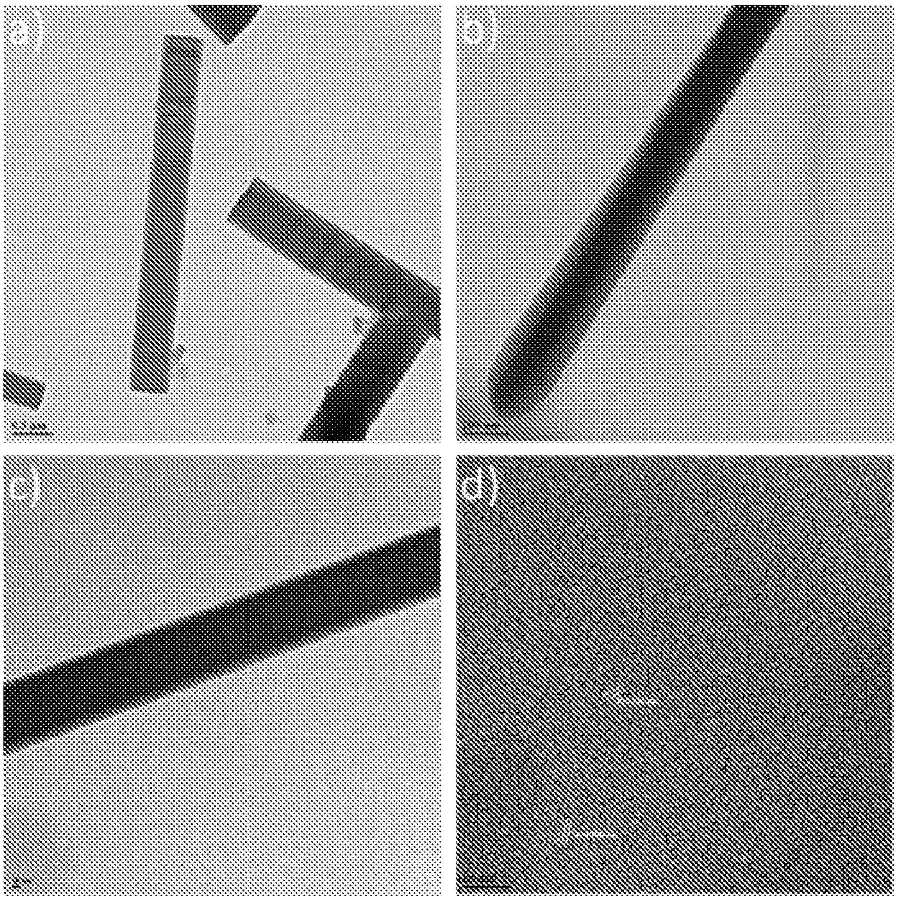


**Fig. 7**

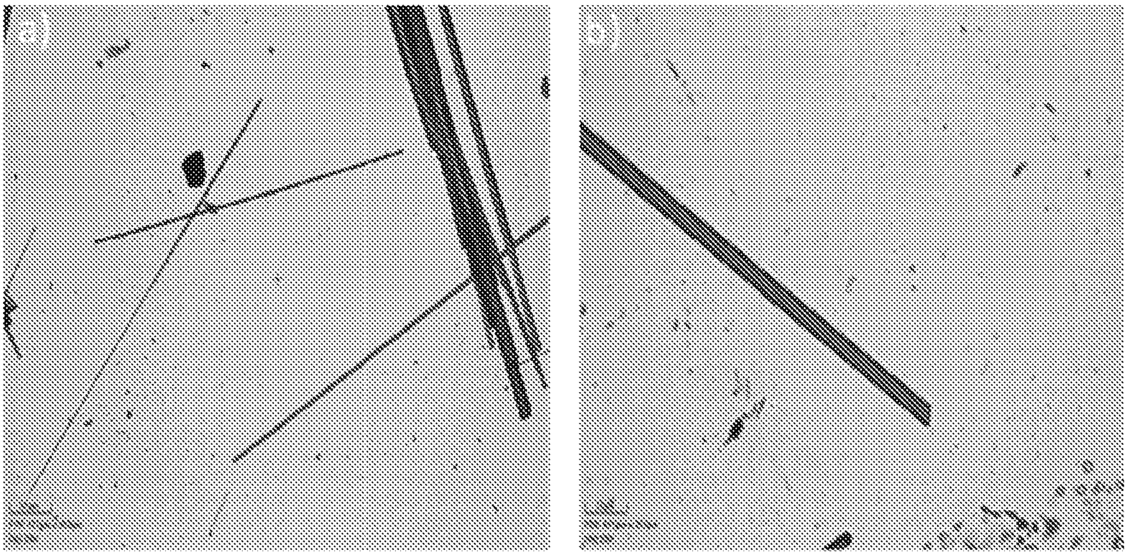


Fig. 8

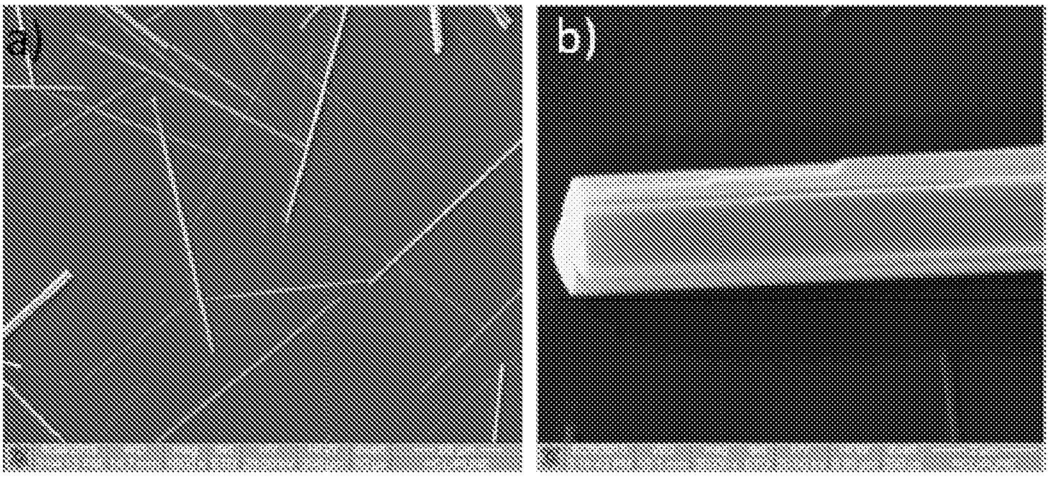


Fig. 9

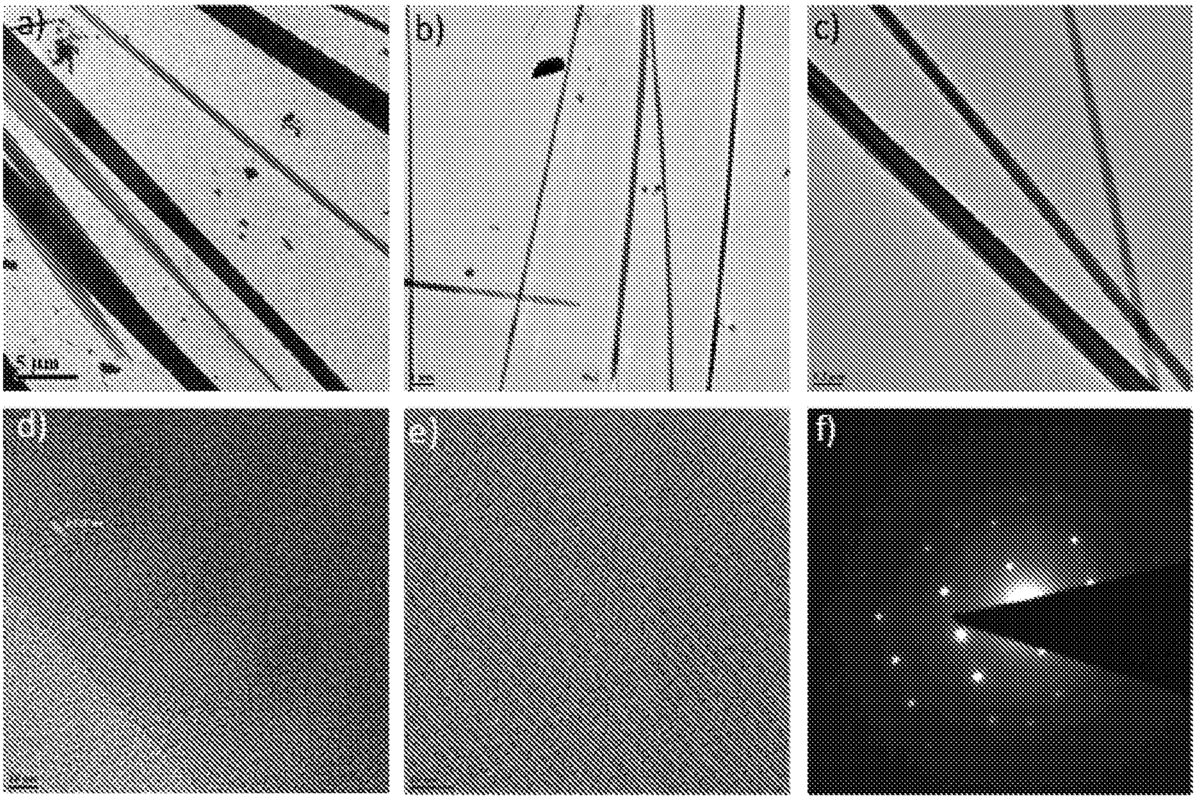


Fig. 10

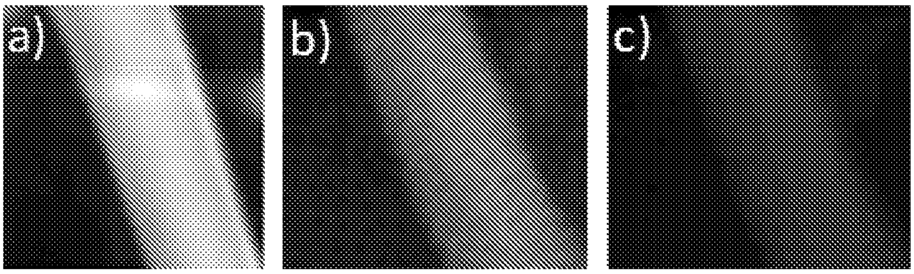


Fig. 11

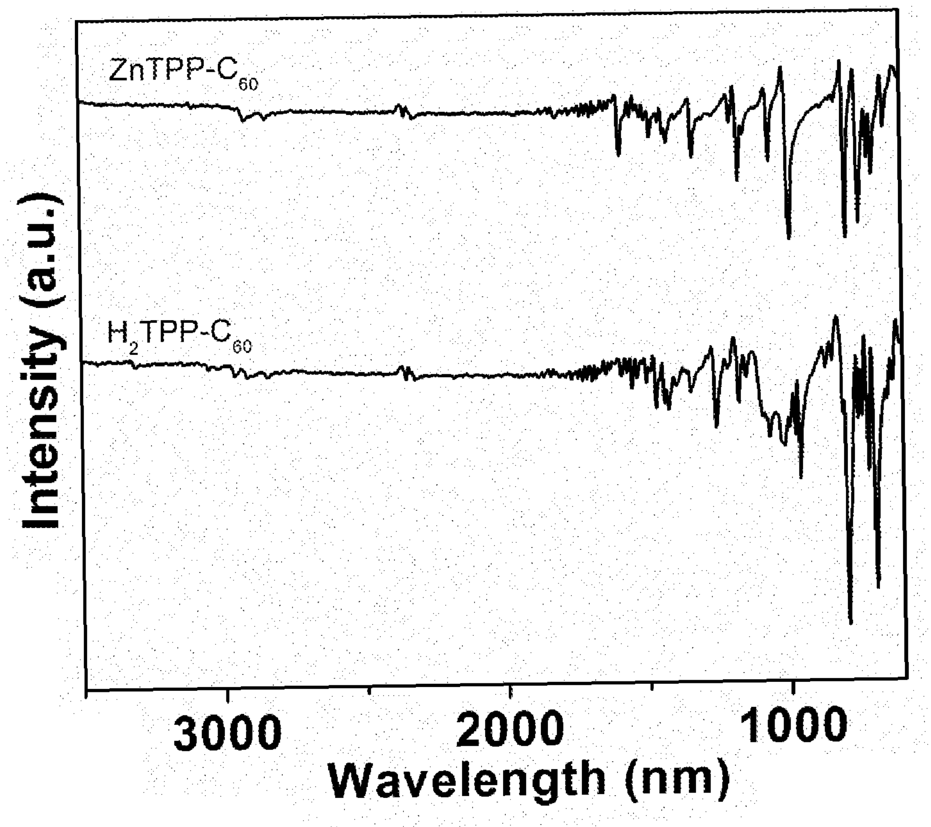
ZnTPP-C60
H2TPP-C60
Intensity (a.u.)
3000
2000
1000
Wavelength (nm)

Fig. 12

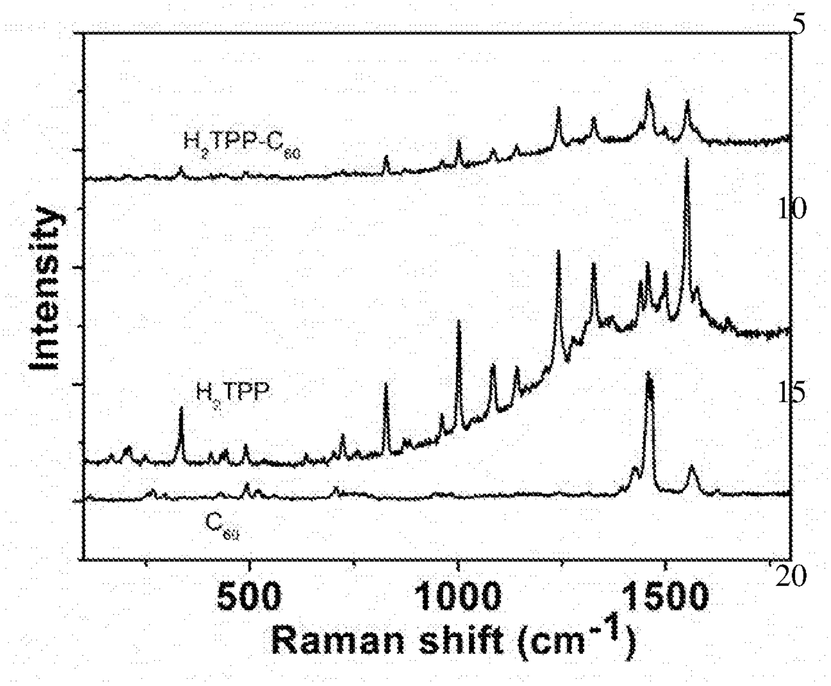
H$_2$TPP-C$_{60}$
H$_2$TPP
C$_{60}$
Intensity
500
1000
1500
Raman shift (cm$^{-1}$)

Fig. 13

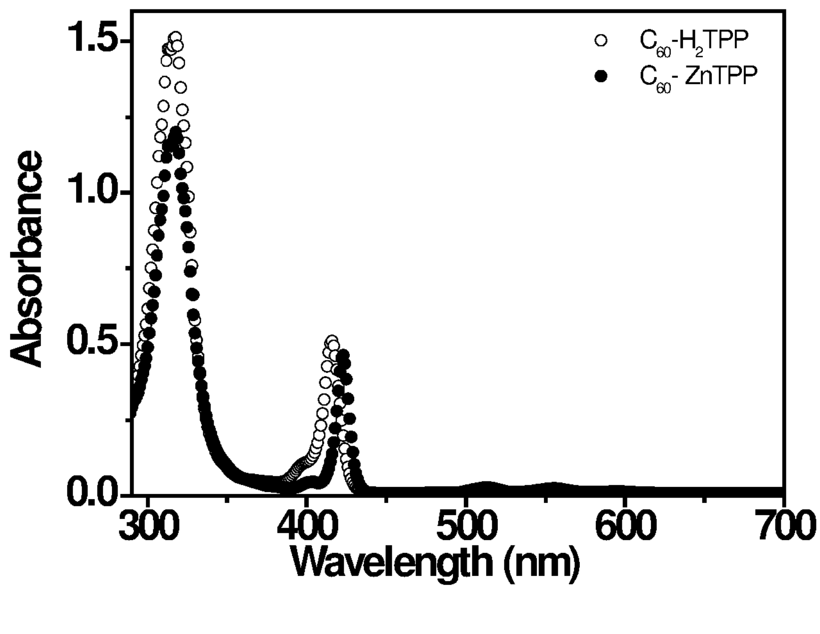
C$_{60}$-H$_2$TPP
C$_{60}$- ZnTPP
Absorbance
1.5
1.0
0.5
0.0
300
400
500
600
700
Wavelength (nm)

Fig. 14

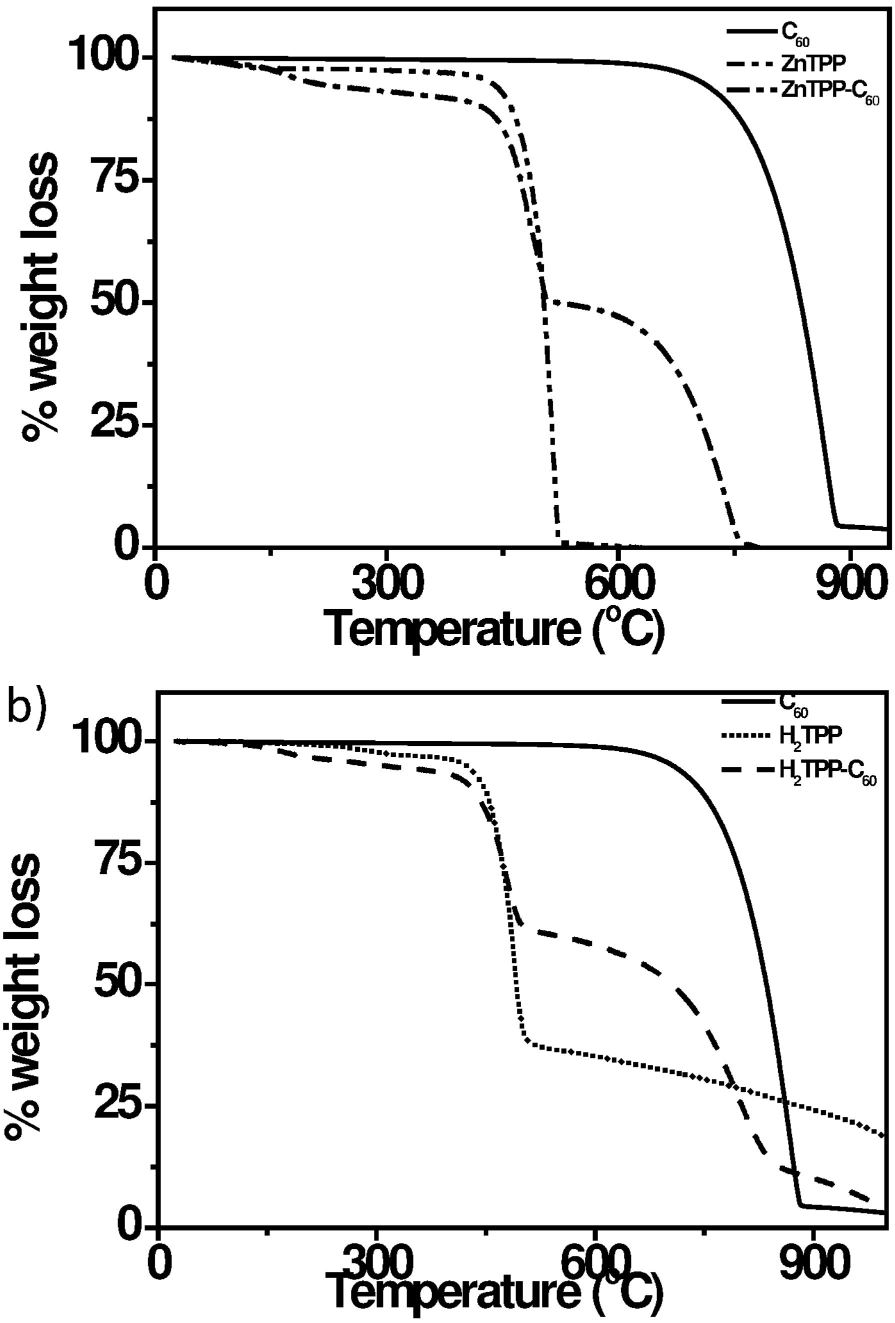

C60
ZnTPP
ZnTPP-C60
% weight loss
100
75
50
25
0
0
300
600
900
Temperature (°C)
b)
C60
H2TPP
H2TPP-C60
% weight loss
100
75
50
25
0
0
300
600
900
Temperature (°C)


Fig. 15

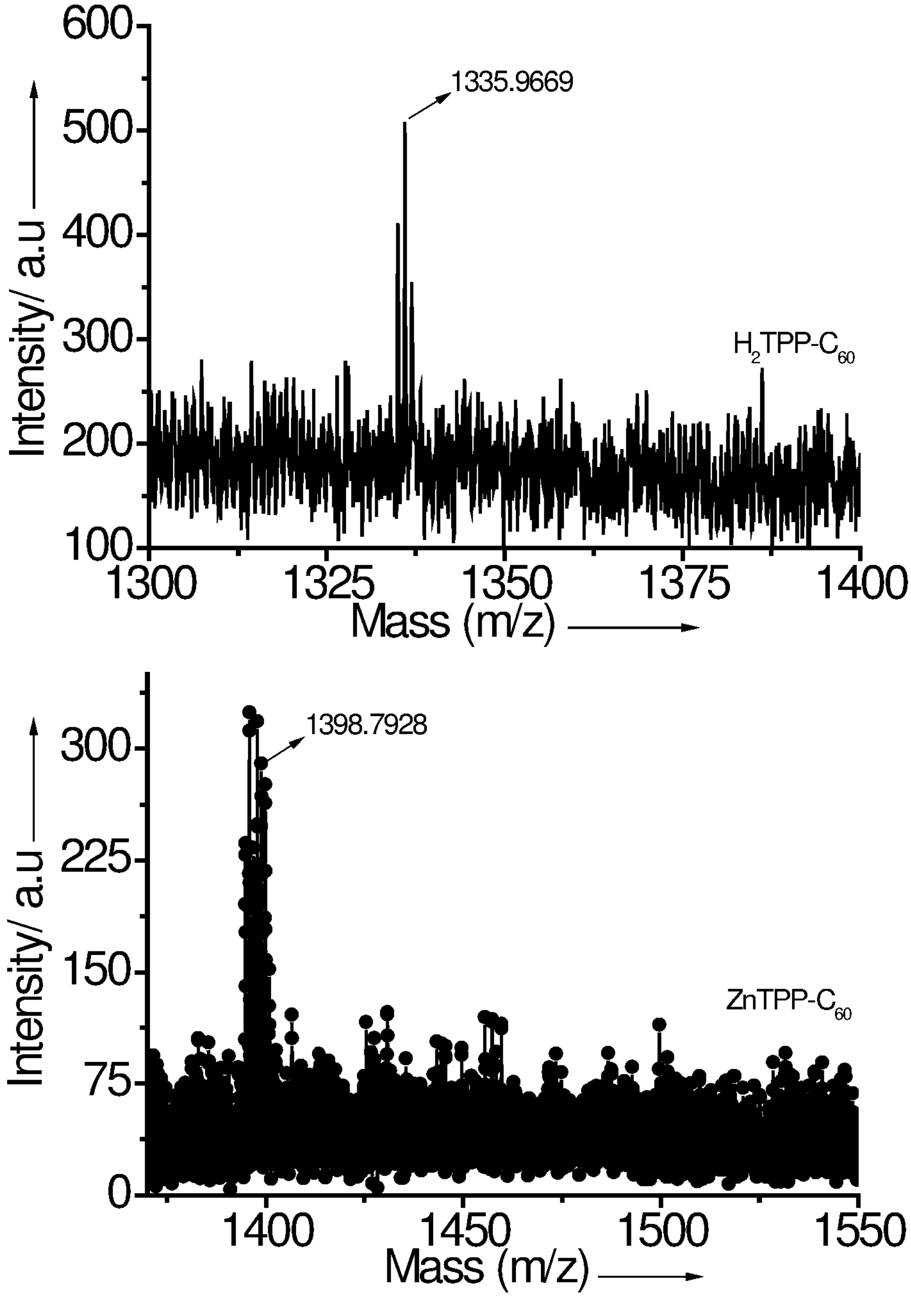
600
500
400
300
200
100
Intensity/ a.u
1335.9669
$H_2TPP-C_{60}$
1300
1325
1350
1375
1400
Mass (m/z)
300
225
150
75
0
Intensity/ a.u
1398.7928
$ZnTPP-C_{60}$
1400
1450
1500
1550
Mass (m/z)

Fig. 16

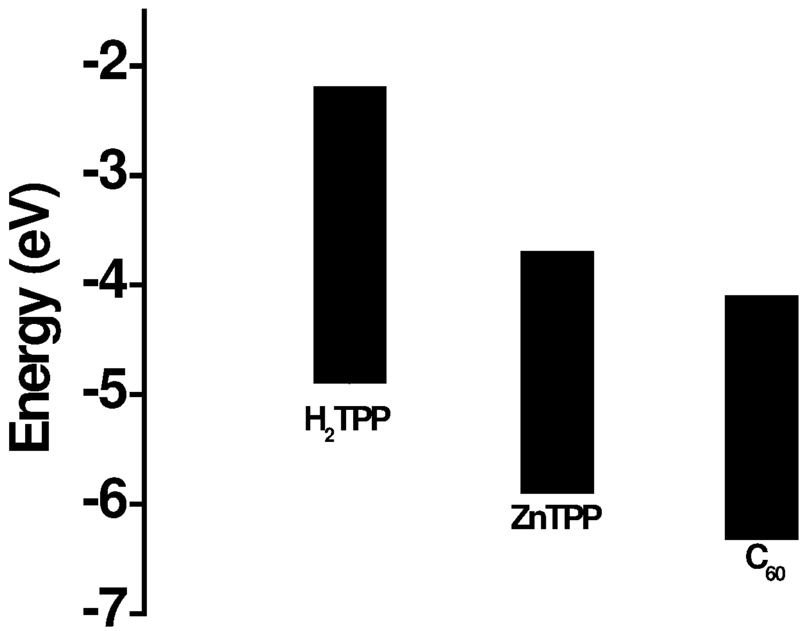


Fig. 17

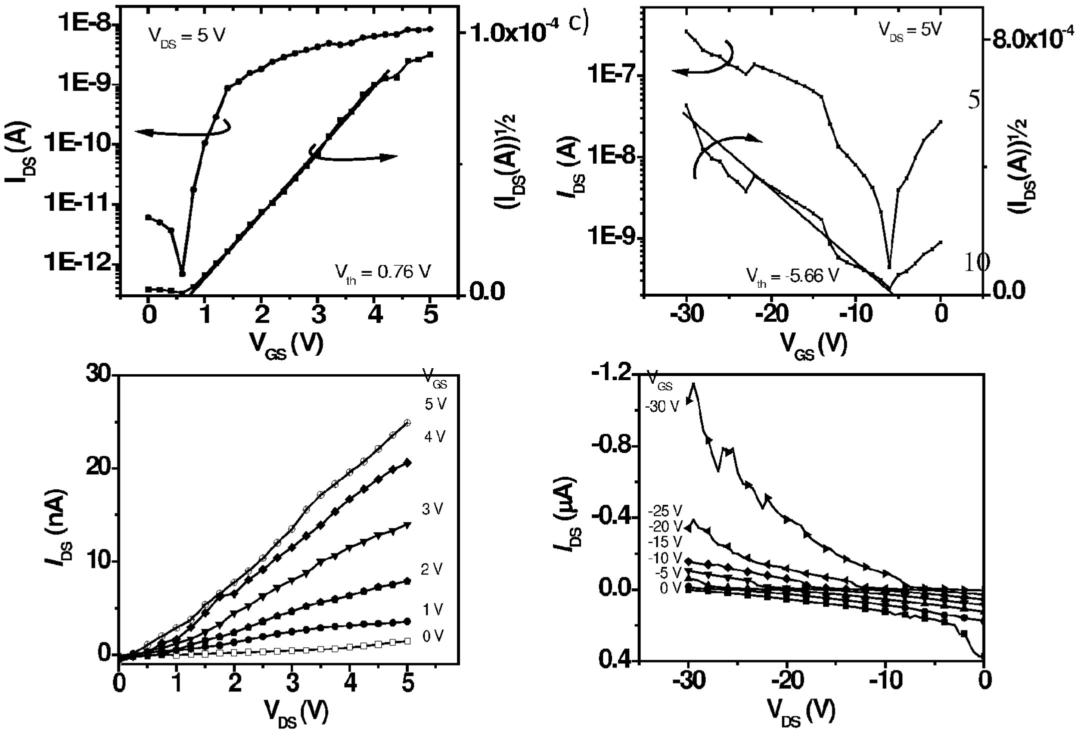
c)
$V_{DS}$ = 5 V
$V_{th}$ = 0.76 V
$I_{DS}$ (A)
$(I_{DS}(A))^{1/2}$
1.0x10$^{-4}$
$V_{GS}$ (V)
$V_{DS}$ = 5V
$V_{th}$ = -5.66 V
8.0x10$^{-4}$
$I_{DS}$ (nA)
$V_{DS}$ (V)
$V_{GS}$
5 V
4 V
3 V
2 V
1 V
0 V
$I_{DS}$ (μA)
-30 V
-25 V
-20 V
-15 V
-10 V
-5 V
0 V

Fig. 18

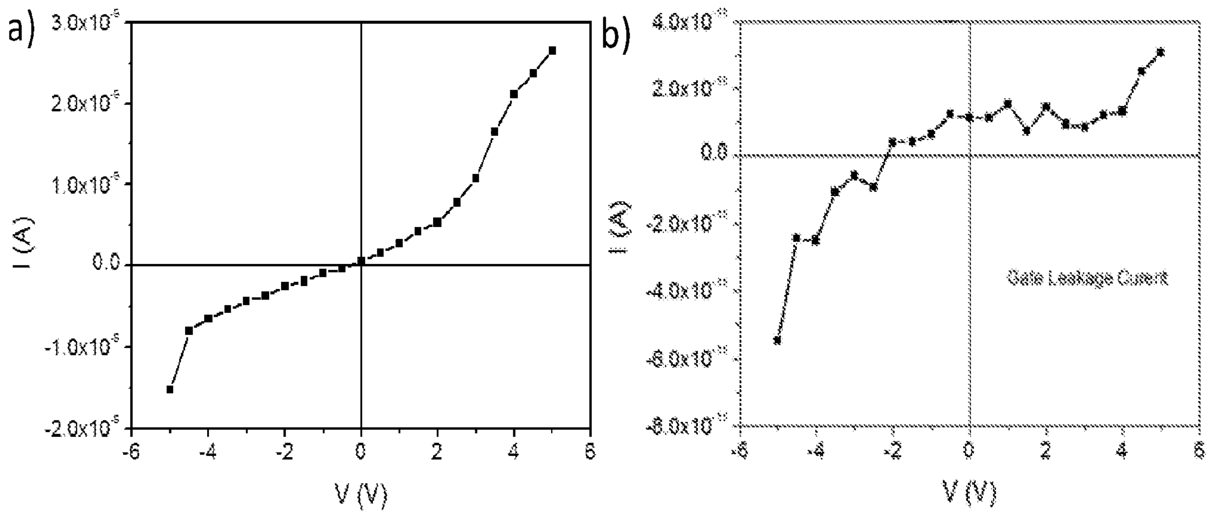
a)
I (A)
V (V)
b)
I (A)
Gate Leakage Curent
V (V)

Fig. 19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **H LI et al.** High-Mobility Field-effect transistors from large-area solution-grown aligned C60 single crystals. *J. Am. Chem. Soc.,* 2012, vol. 134 (5), 2760-2765
- **UJ CASTILLO et al.** Large face to face tetraphenylporphyrin/fullerene nanoaggregates. A DFT study. *Organic Electronics,* 2013, vol. 14 (10), 2617-2627
- **E ITOH et al.** Enhancement of the open-circuit voltage and hole conduction of tetraphenylporphyrin /C60 multilayered photovoltaic device by the insertion of oxide hole collection layers. *Japanese Journal of Applied Physics,* 2011, vol. 50 (1S2
- **T UMEYAMA et al.** Self-organization of porphyrins and fullerenes for molecular photoelectrochemical devices. *Photosynthesis Research,* 2006, vol. 87 (1), 63-71
- **A GARAI et al.** Synthesis, electron transport, and charge storage properties of fullerene-zinc porphyrin hybrid nanodiscs. *RSC Adv.,* 2014, vol. 4, 64119-64127
- **J ZHANG et al.** Fullerene/Sulfur-Bridged Annulene Cocrystals: Two-Dimensional Segregated Heterojunctions with Ambipolar Transport Properties and Photoresponsivity. *J. Am. Chem. Soc.,* 2013, vol. 135 (2), 558-561
- **T WAKAHARA et al.** Fullerene/Cobalt Porphyrin hybrid nanosheets with ambipolar charge transporting characteristics. *J. Am. Chem. Soc.,* 2012, vol. 134 (17), 7204-7206
- **D. KONAREV et al.** New Molecular Complexes of Fullerenes C 60 and C 70 with Tetraphenylporphyrins [M(tpp)], in which M=H2, Mn, Co, Cu, Zn and FeCl. *Chemistry, A European Journal Soc.,* 05 June 2001, 2605-2616
- **ALEKSEY L. LITVINOV et al.** 60]Fullerene Complexes with Supramolecular Zinc Tetraphenylporphyrin Assemblies, Synthesis, Crystal Structures, and Optical Properties. *Crystal Growth & Design.,* 11 August 2005, vol. 5 (5), 1807-1819
- **ANDREY F. MIRONOV.** Synthesis, Properties, and Potential Applications of Porphyrin-Fullerenes. *Macroheterocycles,* 2011, vol. 4 (3), 186-208